# EUROPEAN PATENT SPECIFICATION

(11) **EP 0 731 681 B1**
(45) Date of publication and mention of the grant of the patent: **30.08.2000**
(21) Application number: 95901868.0
(22) Date of filing: 14.11.1994
(51) Int. Cl.: A61F 13/15

(54) **ABSORBENT ARTICLE WITH ASYMMETRIC SHAPE FOR IMPROVED PROTECTION**
SAUGKÖRPER MIT ASYMMETRISCHER FORM FÜR EINEN BESSEREN SCHUTZ
ARTICLE ABSORBANT POURVU D'UNE FORME ASYMETRIQUE OFFRANT UNE MEILLEURE PROTECTION

(30) Priority: 30.11.1993 US 159396
(43) Date of publication of application: 18.09.1996
(73) Proprietor: THE PROCTER & GAMBLE COMPANY, Cincinnati, Ohio 45202 (US)
(72) Inventor: UNGER, Matthew, Eric, Cincinnati, OH 45240 (US); FITSCHER, Alicia, Marie, West Chester, OH 45069 (US)
(74) Representative: Hirsch, Uwe Thomas
(86) International application number: US9413029
(87) International publication number: WO9515139

(56) References cited:
- EP-A- 0 249 405
- EP-A- 0 302 523
- WO-A-90/05514
- WO-A-90/14814
- US-A- 4 608 047
- US-A- 4 758 241

## Description

### FIELD OF THE INVENTION

The present invention relates to an absorbent article as defined in the preamble of claim 1. Such an absorbent article is known from EP-A-0249405.

### BACKGROUND OF THE INVENTION

Absorbent articles, such as sanitary napkins, pantiliners, and incontinence pads, are typically worn in the crotch region of an undergarment. These devices are designed to absorb and retain liquids and other discharges from the human body to prevent body and garment soiling. The aforementioned types of absorbent articles are typically placed in a garment such as the wearer's panty and worn between the wearer's legs, adjacent to the perineal area of the body.

Current overnight, or extra protection products, such as KOTEX Overnite, and STAYFREE Super Long, currently provide some protection from soiling by utilizing a combination of increased product length and thickness (which may include a profiled or varied caliper). These current products, however, are not believed to conform to the body of the wearer as closely as possible, particularly the portions of the product that are adjacent to the rear of the wearer's body. Thus, leakage is possible, particularly at the rear of these products.

A need exists for an absorbent article, particularly an overnight sanitary napkin that provides improved protection from soiling.

It is, therefore, an object of the present invention to provide an absorbent article, such as a sanitary napkin, that provides improved protection from soiling. It is also an object of the present invention to provide a sanitary napkin that fits closer to the wearer's body in the areas where menses and other exudates originate which retains its ability to cover a given area of the wearer's panty when in use.

These objects are solved by an absorbent article as defined in the characterizing portion of claim 1.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:
FIG. 1 is a top plan view of a preferred embodiment of the absorbent article of the present invention.
FIG. 2 is a side view of the absorbent article shown in FIG. 1.
FIG. 3 is a bottom plan view of the absorbent article shown in FIG. 1.
FIG. 4 is an end view of the absorbent article shown in FIG. 1 taken from the first end region.
FIG. 5 is an end view of the absorbent article shown in FIG. 1 taken from the second end region.
FIG. 6 is a top plan view of the absorbent article of the present invention (having a slightly different plan view shape) showing the details of the construction of the same.
FIG. 7 is a longitudinal side view of the absorbent article shown in FIG. 6.
FIG. 8 is a schematic cross-sectional view of the article shown in FIG. 6 taken along line 8-8 of FIG. 6.
FIG. 9 is a bottom plan view of the absorbent article shown in FIG. 6.
FIG. 10 is a perspective view of the absorbent article shown in FIG. 6 in an "in use" configuration.
FIG. 11 is a top-plan view of a preferred embodiment of the absorbent core of the absorbent article of the present invention (with optional border and side flaps shown with a dashed line).
FIG. 12 is a side view of the absorbent core shown in FIG. 11.
FIG. 13 is a bottom plan view of the absorbent core shown in FIG. 11
FIG. 14 is an end view of the absorbent core shown in FIG. 11 taken from one end.
FIG. 15 is an end view of the absorbent core shown in FIG. 11 taken from the other end.

### DETAILED DESCRIPTION OF THE INVENTION

The present invention relates to absorbent articles, such as sanitary napkins, pantiliners, and incontinence pads. More particularly, the present invention relates to asymmetrically-shaped overnight sanitary napkins having a central region with arcuate channels and an enlarged end region which cooperate to provide the sanitary napkin with improved protection against soiling.

The term "absorbent article," as used herein, refers to articles which absorb and contain body exudates. More specifically, the term refers to articles which are placed against or in proximity to the body of the wearer to absorb and contain the various exudates discharged from the body. The term "absorbent article" is intended to include sanitary napkins, pantiliners, and incontinence pads (and other articles worn in the crotch region of a garment). The term "disposable" refers to articles which are intended to be discarded after a single use and preferably recycled, composted, or otherwise disposed of in an environmentally compatible manner. (That is, they are not intended to be laundered or otherwise restored or reused as an absorbent article.)

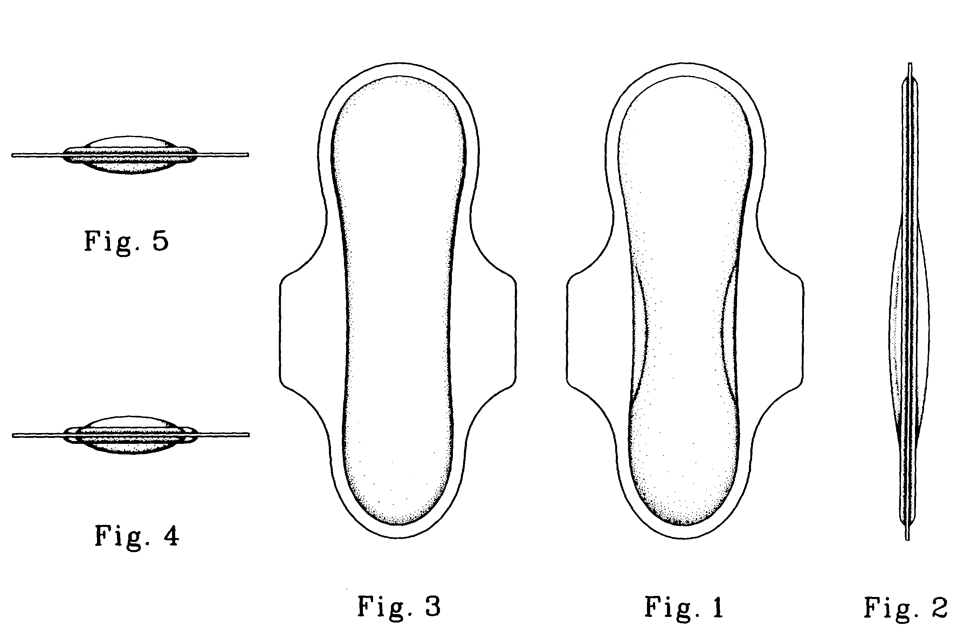

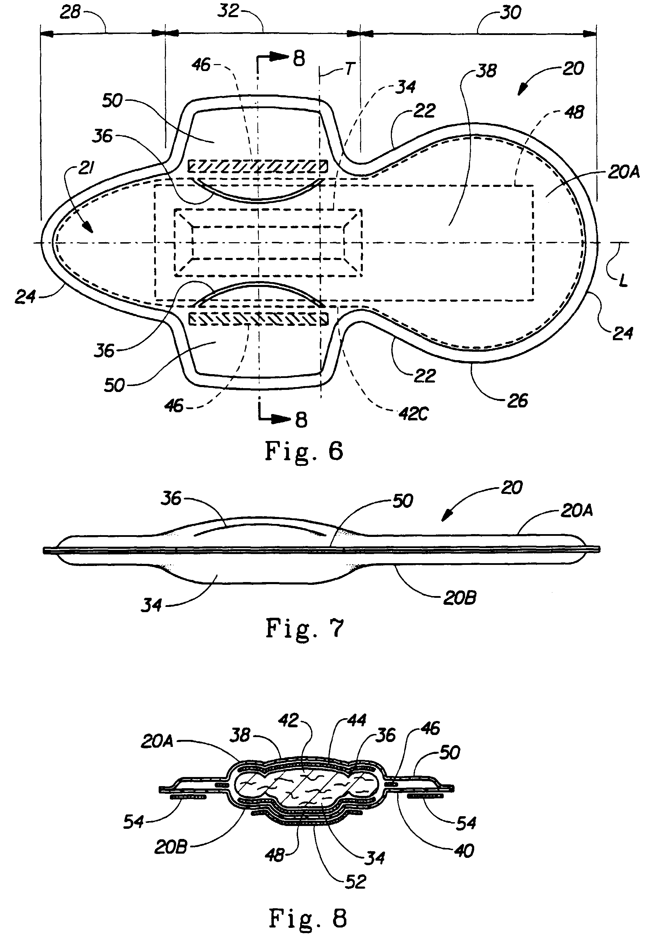

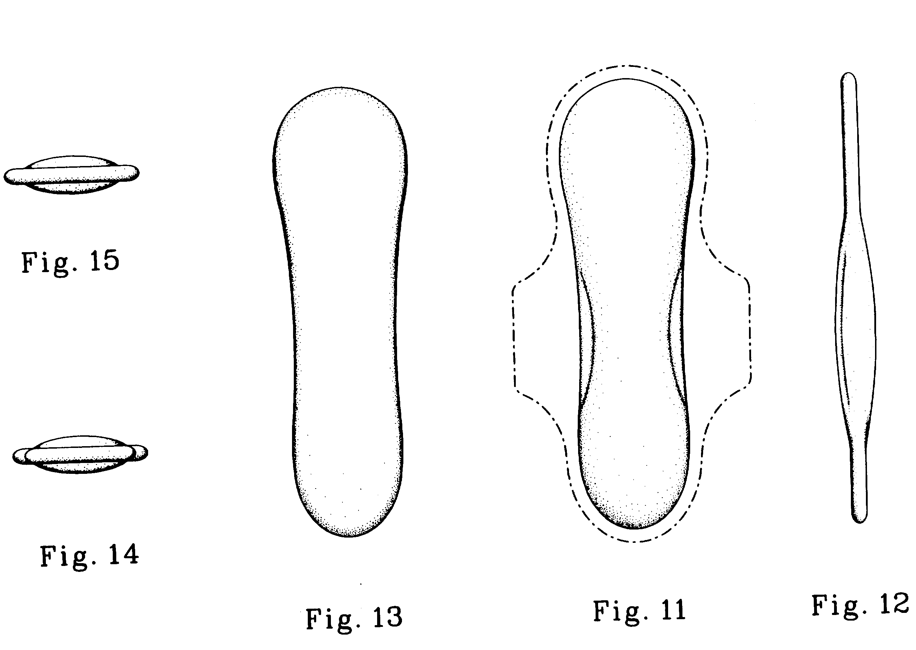

FIGS. 1-5 show a preferred embodiment of the disposable absorbent article of the present invention. FIGS. 6-10 show the details of the construction of such a sanitary napkin (having a slightly different plan view shape). FIGS. 11-15 show a preferred embodiment of an absorbent core for the disposable absorbent article of the present invention. In the preferred embodiments illustrated, the absorbent article is a sanitary napkin designated 20.

The term "sanitary napkin", as used herein, refers to an article which is worn by females adjacent to the pudendal region that is intended to absorb and contain the various exudates which are discharged from the body (e.g., blood, menses, and urine). It should be understood, however, that the present invention is also applicable to other feminine hygiene or catamenial pads such as panty liners (or "pantiliners"), or other absorbent articles such as incontinence pads, or the like.

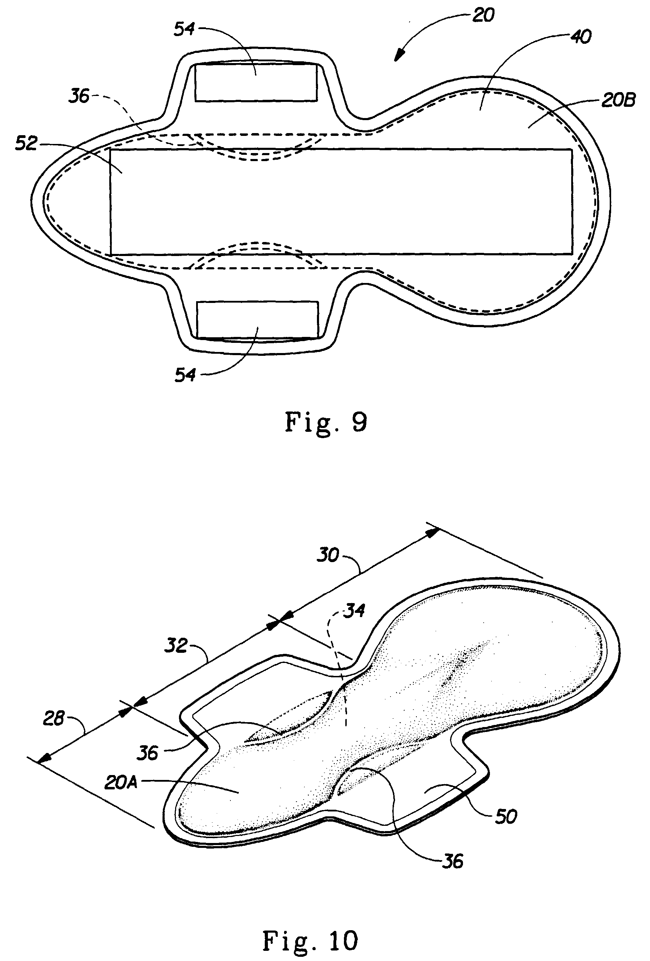

The sanitary napkin 20 has two surfaces, a liquid pervious body-contacting surface or "body surface" 20A and a liquid impervious garment surface 20B. The sanitary napkin 20 is shown in FIG. 6 as viewed from its body surface 20A. The body surface 20A is intended to be worn adjacent to the body of the wearer. The garment surface 20B of the sanitary napkin 20 (shown in FIG. 9) is on the opposite side and is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 6 shows that the sanitary napkin 20 has two spaced apart longitudinal edges 22 and two spaced apart transverse or end edges (or "ends") 24, which together form the periphery 26 of the main body portion 21 of the sanitary napkin 20. The main body portion 21 of the sanitary napkin is that portion of the sanitary napkin exclusive of any optional side flaps 50. The main body portion 21 also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the main body portion. A detailed description of the terms "central region" and "end regions" is contained in U.S. Patent 4,690,680 issued to Higgins on September 1, 1987. The sanitary napkin is preferably worn so that the first end region 28 is placed to the front of the wearer's body and the second end region 30 is placed to the rear of the wearer's body. Thus, the first end region 28 may be referred to as the "front end region" and the second end region may be referred to as the "back end region".

The sanitary napkin 20 (or main body potion thereof) can be of any thickness, including relatively thick, relatively thin, or even very thin. The embodiment of the sanitary napkin 20 shown in Figures 1-10 of the drawings is intended to be an example of a relatively thick overnight, extra protection sanitary napkin having a minimum caliper of about 8 mm in the end regions and a maximum caliper of about 21 mm in the central region 32. These calipers are measured under a pressure of 0.1 psi using a circular comparator foot with a 1 square inch area. It should be understood, however, when viewing these figures the number of layers of material shown may cause the sanitary napkin 20 to appear much thicker than it actually is, particularly in cross-section. The sanitary napkin 20 shown in FIGS. 1-10 is preferably profiled from front to back and from one longitudinal edge to the other edge so that it is thicker in the center. The central region 32 of the sanitary napkin 20 is provided with a pair of longitudinally-oriented arcuate embossed channels 36.

FIG. 8 is a cross-sectional view of the sanitary napkin 20 taken along secton line 8-8 in FIG. 6. FIG. 8 shows the individual components of the sanitary napkin 20 of the present invention. The sanitary napkin shown in FIG. 8 generally comprises at least three primary components. These include a liquid pervious topsheet 38, a liquid impervious backsheet 40, and an absorbent core 42 positioned between the topsheet 38 and the backsheet 40. The faces (or sides) and edges of the components of the sanitary napkin can be designated by the following reference number convention. The side of the component facing the wearer's body can be designated by the number of the component and a reference letter "A". The side facing the wearer's undergarments can be designated by the number of the component and the letter "B". The side and end edges can be designated by the number of the component and the reference letters "C" and "D", respectively.

The topsheet, backsheet, and absorbent core can comprise any types of materials known in the art as being suitable for such components of an absorbent article. Some suitable topsheets, backsheets, and absorbent cores are described in the patents incorporated by reference herein. Some particularly suitable topsheets are described in U.S. Patent 4,342,314 issued to Radel, et al. and U.S. Patent 4,463,045 issued to Ahr, et al. In the preferred embodiment shown, the topsheet comprises a formed film made under both of the above patents, the absorbent core 42 and the thicker portion (or "profiled section") 34 of the core are comprised of airfelt, and the backsheet 40 is a liquid impervious film.

Figure 8 also shows arcuate embossed channels 36 in the absorbent core 42 and the relative depth of the same. The channels 36 are shown in a preferred location next to the profiled section 34 of the absorbent core 42. The thicker portion 34 of the core 42 is disposed longitudinally and laterally between the arcuate channels 36. The embossed channels 36 preferably extend all the way to the longitudinal side edges 42C of the absorbent core 42. In other embodiments, however, they need not. While sanitary napkins having embossed channels are known (e.g., U.S. Patent 4,059,114 issued to Richards on November 22, 1977, and U.S. Patent 4,655,759 issued to Romans-Hess, et al. on April 7, 1987), such channels are not believed to have been utilized to conform the sanitary napkins disclosed in these patents to the wearer's body in the same manner described herein. Further, since the channels in the preferred sanitary napkin embodiment of the present invention extend all the way to the side edges of the core, the sanitary napkin of the present invention does not form an "occlusive volume" as described in the Romans-Hess patent (to the extent that the meaning of that term can be understood).

The topsheet, backsheet, and absorbent core may be assembled in a variety of well known configurations. Preferred configurations into which the sanitary napkin of the present invention can be generally assembled are described in U.S. Patents 4,950,264, 5,009,653 issued to Osborn, U.S. Patent 4,425,130 issued to DesMarais, U.S. Patents 4,589,876 and 4,687,478 issued to Van Tilburg, and U.S. Patent 5,234,422 issued to Sneller, et al. Figures 6-10 show a preferred embodiment of the sanitary napkin 20 in which the topsheet 38 and the backsheet 40 have length and width dimensions generally larger than those of the absorbent core 42. The topsheet 38 and backsheet 40 extend beyond the edges of the absorbent core 42 to thereby form not only portions of the periphery 26 but also side flaps 50. The topsheet 38 is attached to the absorbent core 42 by a core bonding adhesive layer 44. This adhesive layer improves fluid acquisition by keeping underlying absorbent material in contact with the topsheet. It also helps maintain the presence of two longitudinal arcuate channels 36. There is another application of adhesive 46 in the side flaps 50 near the longitudinal edges 42C of the core 42 which aids in core definition. This adhesive layer helps keep the core material inside the center of the absorbent article and reduces blousing between the topsheet 38 and backsheet 40.

FIG. 9 shows the fasteners used to attach the sanitary napkin 20 to the wearer's panties. The fasteners comprise three patches of adhesive adhered to the backsheet 40 of the sanitary napkin 20. The panty fastening adhesive 52 adheres the main body portion 21 of the sanitary napkin to the crotch area of the wearer's undergarment to help keep the product in place. The other two adhesive patches are located on the side flaps, or wings 50, of the sanitary napkin 20. These two patches are the flap adhesives 54. These are present on the side flaps to help them adhere to the undersides of the wearer's undergarment during use and to help keep the product in place to improve fit. These adhesive patches are preferably covered by one or more cover strips or release papers. In a particularly preferred embodiment, the flaps 50 are folded over the topsheet and the adhesive patches 54 thereon are bridged by a release strip, and the panty fastening adhesive 52 is covered by a releasable wrapper that also serves as a package for the sanitary napkin (the latter being described in U.S. Patent 4,556,146 issued to Swanson, et al.)

The sanitary napkin 20 is asymmetrically shaped so that the second end region 30 is larger than the first end region 28. A preferred embodiment is asymmetrically shaped such that the second end region 30 has both a larger width (or a larger diameter, if the end regions are circular) and a longer length than the first end region 28. The ratio of the widths, or diameters, of the end regions preferably ranges from between about 1:1 and about 2:1. Preferably, the ratio of the width of the second end region 30 to the first end region 28 is about 1.5:1. The ratio of the length of the second end region 30 to the first end region 28 preferably ranges from between about 1:1 and about 2:1. Preferably, the ratio of the length of the second end region 30 to the first end region 28 is about 1.4:1. Preferably, the ratio of the length of the central region 32 to the second end region 30 is 1.1:1. The absorbent article is preferably worn by the wearer so that the second end region is placed to the rear of the wearer's body to provide the best perineal and gluteal fit. The first end region 28, second end region 30, and central region 32 can have any suitable plan view shape. In the preferred embodiment shown in FIGS. 1-5, the sanitary napkin 20 has an asymmetrical dog bone plan view shape. In the preferred embodiment shown in Figs. 6-10, the plan view shape of the sanitary napkin resembles the shape of a lightbulb (with two rounded end regions).

In a preferred embodiment, the absorbent core 42 is profiled from front to back (longitudinally) and from side to side (laterally). Preferably, the first end region 28 and the second end region 30 have the same thickness, while the central region 32 comprises the thickest portions of the sanitary napkin. The height ratio of the profile preferably ranges from about 1:1 to about 3:1, and more preferably is about 2.6:1. This profile enables the sanitary napkin to conform better to the wearer's body. A preferred embodiment has a total core length ranging between about 290 and 317 mm. A length shorter than about 290 mm typically does not extend far enough into the gluteal area to provide improved protection. A length longer than about 317 mm generally extends too far beyond the gluteal groove for all of the second end region to conform to the wearer's buttocks leading to poor gluteal fit.

The preferred embodiment shown in the drawings has a pair of longitudinally-oriented arcuate embossed channels 36 in the central region 32 of the sanitary napkin. The profiled section 34 of the core, as mentioned above, lays between the embossed channels 36 in the central region 32 of the sanitary napkin. The arcuate channels 36 assist the central region 32 of the sanitary napkin in assuming a W-shaped cross-sectional configuration when the napkin is compressed by the wearer's thighs, which allows improved contact with the wearer's perineal area. This W-shaped configuration is formed by the sides compressing upwards and inwards and the center buckling upward into a convex upward configuration, with the densified region provided by the channels forming the fold lines.

FIG. 10 is a perspective view of the sanitary napkin 20 depicted in FIGS. 6-9 showing a preferred in-use configuration of the napkin 20. FIG. 10 shows the W-shape configuration of the central region 32 and the inverted V-shape (or inverted U-shape) in the second end region 30 of the sanitary napkin 20. Figure 10 shows that as the central region 32 transitions to the second end region 30 (at the ends of the embossed channels 36), the W-shape configuration is transformed into an inverted V-shape configuration. The absence of the channels in the second end region 30 eliminates the folding of the napkin into the "legs" of the W-shape configuration. The inverted V-shape configuration retains the raised central portion of the W-shape configuration to improve gluteal fit. The second end region 30 is thus able to closely fit into the crevice between the wearer's buttocks (that is, in the gluteal groove) for improved protection from end soiling. This occurs while the second end region 30 maintains its area coverage due to size of the same and the extensive panty fastening adhesive 52 (described below).

The sanitary napkin 20 preferably has sufficient panty fastening adhesive 52 to keep the second end region 30 in place and maintain its area coverage (amount of the panties covered by the second end region 30). The panty fastening adhesive 52 is preferably at least about 50% as long as the absorbent core 42, but is preferably no greater than about 100% of the length of the core. The preferred length of the panty fastening adhesive 52 is between about 75% and about 90% the length of the absorbent core 42. The width of the panty fastening adhesive 52 is preferably at least about 25% of the width of the portion of the absorbent core lying in the first end region 28, but should be no wider than about 100% the width of the portion of the core in the first end region 28. The preferred width of adhesive is between about 70% and about 80% the width of the first end region core width.

The preferred embodiment shown in the drawings also has a pair of wings 50 which extend laterally outward from the central region 32, beginning at the transition between the first end region 28 and the central region 32, and ending at the transition between the second end region 30 and the central region 32. These wings 50, which have panty fasteners 54 thereon, adhere to the underside of the wearer's undergarment. The wings 50 serve to keep the sanitary napkin 20 in place during wear. Keeping the sanitary napkin in place prevents skewing of the napkin in the panty, and undesirable bunching. The wings 50 may also assist the central region 32 in forming into the desired W-shaped configuration, and the second end region 30 in forming the desired inverted V-shaped configuration.

Thus, in a particularly preferred embodiment, the present invention uses an asymmetrical shape and structure to provide added protection in combination with embossed channels, wings, longitudinal and lateral profiling and extensive adhesive that synergistically enhance the structure's ability to provide improved fit in the perineal area and crevice between the buttocks (i.e., "gluteal groove") while maintaining area coverage.

## Claims

1. An absorbent article having a pair of longitudinal edges (22) a pair of transverse edges (24), a first end region (28), a second end region (30), and a central region (32) disposed between said end regions, said absorbent article comprising:
a liquid pervious topsheet (38);
a liquid impervious backsheet (40);
an absorbent core (42) positioned between said topsheet and said backsheet;
said absorbent article has a pair of arcuate channels (36), said first end (28) region has a width defined between said longitudinal edges (22), said second end region (30) has a width defined between said longitudinal edges (22) characterised in that said channels are in said absorbent core within said central portion and the ratio of the width of said second end region to the width of said first end region ranges from greater than or equal to 1.5:1 up to 2:1, wherein said central region (32) of said absorbent article assumes a convex upward cross-sectional W shaped configuration when laterally compressed and said second end (30) region assumes an inverted V-shaped configuration beyond said channels (36) when said absorbent article is worn.

2. The absorbent article of Claim 1 wherein said absorbent core (42) is profiled so that said central region (32) has a caliper that is greater than the caliper of said end regions.

3. The absorbent article of Claim 2 wherein said absorbent core (42) comprises a thicker portion located between said arcuate channels (36).

## Patentansprüche

1. Ein absorbierender Artikel mit einem Paar Längsrändern (22), einem Paar Querrändern (24), einem ersten Endbereich (28), einem zweien Endbereich (30) und einem Mittelbereich (32), welcher zwischen den genannten Endbereichen angeordnet ist, wobei der genannte absorbierende Artikel umfaßt:
ein flüssigkeitsdurchlässiges Deckblatt (38);
ein flüssigkeitsundurchlässiges Rückenblatt (40);
einen absorbierenden Kern (42), welcher zwischen dem genannten Deckblatt und dem genannten Rückenblatt positioniert ist;
wobei der genannte absorbierende Artikel ein Paar bogenförmiger Kanäle (36) aufweist, der genannte erste Endbereich (28) eine Breite aufweist, welche zwischen den genannten Längsrändern (22) definiert ist, der genannte zweite Endbereich (30) eine Breite aufweist, welche zwischen den genannten Längsrändern (22) definiert ist, dadurch gekennzeichnet, daß die genannten Kanäle im genannten absorbierenden Kern innerhalb des genannten Mittelabschnitts sind und das Verhältnis der Breite des genannten zweiten Endbereichs zur Breite des genannten ersten Endbereichs im Bereich von mehr als oder gleich 1,5:1 bis zu 2:1 liegt, wobei der genannte Mittelbereich (32) des genannten absorbierenden Artikels eine konvexe aufwärts gerichtete W-förmige Querschnittskonfiguation annimmt, wenn er seitlich komprimiert ist, und der genannte zweite Endbereich (30) eine umgekehrte V-förmige Konfiguration über die genannten Kanäle (36) hinaus annimmt, wenn der genannte absorbierende Artikel getragen wird.

2. Der absorbierende Artikel nach Anspruch 1, bei welchem der genannte absorbierende Kern (42) so profiliert ist, daß der genannte Mittelbereich (32) eine Abgreifhöhe aufweist, welche größer als die Abgreifhöhe der genannten Endbereiche ist.

3. Der absorbierende Artikel nach Anspruch 2, bei welchem der genannte absorbierende Kern (42) einen zwischen den genannten bogenförmigen Kanälen (36) angeordneten dickeren Abschnitt umfaßt.

## Revendications

1. Article absorbant ayant une paire de bords longitudinaux (22), une paire de bords transversaux (24), une première région d'extrémité (28), une deuxième région d'extrémité (30) et une région centrale (32) placée entre lesdites régions d'extrémité, ledit article absorbant comprenant :
une feuille de dessus (38) perméable aux liquides ;
une feuille de fond (40) imperméable aux liquides ;
une âme absorbante (42) placée entre ladite feuille de dessus et ladite feuille de fond ;
ledit article absorbant a une paire de canaux incurvés (36), ladite première région d'extrémité (28) a une largeur définie entre lesdits bords longitudinaux (22), ladite deuxième région d'extrémité (30) a une largeur définie entre lesdits bords longitudinaux (22), caractérisé en ce que lesdits canaux sont dans ladite âme absorbante à l'intérieur de ladite partie centrale, et le rapport entre la largeur de ladite deuxième région d'extrémité et la largeur de ladite première région d'extrémité est compris dans une plage allant d'un rapport supérieur ou égal à 1,5 : 1 à un rapport de 2 : 1 ; dans lequel ladite région centrale (32) dudit article absorbant prend une configuration en forme de W de section transversale convexe vers le haut lorsqu'elle est comprimée latéralement, et ladite deuxième région d'extrémité (30) prend une configuration en forme de V renversé au-delà desdits canaux (36) lorsque ledit article absorbant est porté.

2. Article absorbant selon la revendication 1, dans lequel ladite âme absorbante (42) est profilée afin que ladite région centrale (32) présente une épaisseur qui est plus grande que celle desdites régions d'extrémité.

3. Article absorbant selon la revendication 2, dans lequel ladite âme absorbante (42) comprend une partie plus épaisse située entre lesdits canaux incurvés (36).
